# EUROPÄISCHE PATENTSCHRIFT

(11) **EP 1 133 470 B1**
(45) Veröffentlichungstag und Bekanntmachung des Hinweises auf die Patenterteilung: **21.05.2003**
(21) Anmeldenummer: 99962150.1
(22) Anmeldetag: 22.11.1999
(51) Int. Cl.: C07C 303/16, C07C 309/04

(54) **VERFAHREN ZUR HERSTELLUNG VON ALKANSULFONSÄUREN**
METHOD OF PRODUCING ALKANE SULFONIC ACID
PROCEDE POUR PREPARER DES ACIDES SULFONIQUES D'ALCANE

(30) Priorität: 25.11.1998 DE 19854428
(43) Veröffentlichungstag der Anmeldung: 19.09.2001
(73) Patentinhaber: BASF AKTIENGESELLSCHAFT, 67056 Ludwigshafen (DE)
(72) Erfinder: EIERMANN, Matthias, D-67117 Limburgerhof (DE); TRAGUT, Christian, D-67157 Wachenheim (DE); EBEL, Klaus, D-68623 Lampertheim (DE)
(74) Vertreter: Isenbruck, Günter, Dr.
(86) Internationale Anmeldenummer: EP9908994
(87) Internationale Veröffentlichungsnummer: WO00031027

(56) Entgegenhaltungen:
- WO-A-98/34914
- US-A- 2 697 722
- US-A- 2 727 920

## Beschreibung

Die Erfindung betrifft ein Verfahren zur Herstellung von Alkansulfonsäuren.
Alkansulfonsäuren werden in einer Reihe technischer Anwendungen eingesetzt. Langkettige Alkansulfonsäuren weisen z.B. Tensideigenschaften auf, während kurzkettige, wie Methansulfonsäure, z.B. als Hilfschemikalien zur galvanischen Abscheidung unedler Metalle wie Zinn oder Blei in der Verzinnung von Leiterplatten für die Elektronik oder in der Herstellung von Weißblech eingesetzt werden können.

In der Literatur sind eine Reihe von Verfahren zur Herstellung von Alkansulfonsäuren beschrieben. Dabei dienen insbesondere Alkylmercaptane oder Dialkyldisulfide als Ausgangsmaterialien, die üblicherweise durch die Umsetzung von Schwefelwasserstoff mit Alkoholen hergestellt werden. Die Oxidationsreaktion der Alkylmercaptane oder der Dialkyldisulfide zur entsprechenden Alkansulfonsäure kann durch verschiedene Oxidationsmittel erreicht werden. So können als Oxidationsmittel Wasserstoffperoxid, Chlor, Dimethylsulfoxid oder Gemische aus Dimethylsulfoxid und Iodwasserstoffsäure sowie die elektrochemische Oxidation eingesetzt werden.

In WO 98/34914 ist eine Oxidation von Mercaptanen und/oder Dialkyldisulfiden mit Br₂ zu Alkansulfonsäuren beschrieben. Das Br₂ wird zur einfacheren Handhabbarkeit vorzugsweise aus HBr gewonnen. Die Oxidation von HBr zu Br₂ kann mit Sauerstoff in Gegenwart katalytischer Mengen Salpetersäure oder mit Salpetersäure selbst als Oxidationsmittel erfolgen. Die bei der Oxidation von HBr mit Salpetersäure entstehenden Stickoxide werden mit Sauerstoff zu Salpetersäure reoxidiert. Um eine Überoxidation der im Verfahren anwesenden Schwefelverbindungen zu Schwefelsäure zu vermeiden, können die Oxidation von HBr zu Br₂ und die Oxidation der Mercaptane und/oder Dialkyldisulfide mit Br₂ in getrennten Reaktoren durchgeführt werden.

Ein weiterer Weg zur Herstellung der Alkansulfonsäuren ist die Oxidation der Alkylmercaptane oder Dialkyldisulfide mit Sauerstoff in Gegenwart von Stickoxiden bzw. Salpetersäure. Die Oxidation mit Sauerstoff in Gegenwart von Salpetersäure wird z.B. in US 2,697,722 und US 2,727,920 beschrieben.

Diese Veröffentlichungen betreffen die Oxidation von Alkylmercaptanen oder Polysulfiden (wie Dialkyldisulfiden) mit in Salpetersäure absorbiertem Sauerstoff. Das Alkylmercaptan bzw. das Polysulfid wird schrittweise zu der gewünschten Alkansulfonsäure oxidiert. Während der Oxidation entstehen Gemische aus Stickstoffmonoxid, Stickstoffdioxid und Lachgas. Das Stickstoffmonoxid und das Stickstoffdioxid werden durch den in der Salpetersäure absorbierten Sauerstoff zu reinem Stickstoffdioxid bzw. zu Salpetersäure umgesetzt, die wieder zur Bildung von Alkansulfonsäuren bereitstehen. Das Lachgas wird ausgeschleust. Ein Nachteil in diesem Verfahren ist der hohe Anteil an gebildetem Lachgas, das als sogenanntes "Treibhausgas" ähnlich wie halogenierte Methane und Ethane zu ökologischen Problemen führt und daher in einer technischen Anlage aufwendig aus dem Abgasstrom abgetrennt werden muß. Des weiteren enthalten die Abgase gleichzeitig größere Mengen an Stickstoff- und Schwefelverbindungen, die ebenfalls aurwendig entfernt werden müssen.

Die Reaktionstemperaturen liegen bei diesen Umsetzungen üblicherweise im Bereich zwischen 25 und 70 °C. Bei diesen Temperaturen wird jedoch kein Vollumsatz zur Alkansulfonsäure erreicht. So bleibt beispielsweise bei der Umsetzung zur Methansulfonsäure die Reaktion bei diesen Reaktionsbedingungen zum Teil auf der Stufe des Zwischenprodukts Methanthiosulfonsäure-S-methylester stehen. Dieses Zwischenprodukt ist eine instabile Verbindung, die bereits ab 90 °C Schwefeldioxid abspaltet und sich bei 170 °C spontan und stark exotherm zersetzt.

Es besteht daher die Aufgabe, ein wirtschaftlich attraktives Verfahren bereitzustellen, das die Herstellung von Alkansulfonsäuren in hoher Reinheit und in guten Ausbeuten ermöglicht, und die Bildung von Lachgas nahezu vollständig unterdrückt.

Diese Aufgabe wird durch ein Verfahren zur Herstellung von Alkansulfonsäuren gelöst, umfassend die folgenden Schritte:
(a) Oxidation von Alkylmercaptanen und/oder Dialkyldisulfiden und/oder Dialkylpolysulfiden mit drei bis neun Schwefelatomen mit Salpetersäure unter Bildung von Alkansulfonsäuren, Wasser, Stickoxiden sowie weiteren Nebenprodukten,
(b) Regenerierung der aus Schritt (a) erhaltenen Stickoxide mit Sauerstoff zu Salpetersäure und Rückführung der Salpetersäure in Schritt (a).
Das erfindungsgemäße Verfahren ist dann dadurch gekennzeichnet, daß die Schritte (a) und (b) in voneinander getrennten Reaktionsräumen durchgeführt werden.

Als Bruttoreaktion wird demnach eine Oxidation des Alkylmercaptans bzw. des Dialkyldisulfids mit (Luft-)Sauerstoff durchgeführt.

Bei den in Schritt (a) entstehenden Stickoxiden handelt es sich um niedrig oxidierte Stickstoffverbindungen (NO/NO₂-Gemische), die in Schritt (b) zu reiner oder Stickstoffdioxid enthaltender Salpetersäure reoxidiert werden. Die in dem erfindungsgemäßen Verfahren eingesetzte Salpetersäure kann demgemäß reine oder Stickstoffdioxid enthaltende Salpetersäure sein.

Die räumliche Trennung der Oxidation von Mercaptanen und/oder Dialkyldisulfiden und/oder Dialkylpolysulfiden mit drei bis neun Schwefelatomen zu Alkansulfonsäure (Schritt (a)) und der Regenerierung der Stickoxide (Schritt (b)) ist deshalb vorteilhaft, weil beide Reaktionsschritte, Schritt (a) und Schritt (b), getrennt voneinander unter optimalen Reaktionsbedingungen durchgeführt werden können. Dadurch kann die Bildung von Lachgas nahezu vollständig unterdrückt werden, und es können sehr gute Ausbeuten an Alkansulfonsäuren erzielt werden.

Vorzugsweise wird das erfindungsgemäße Verfahren kontinuierlich durchgerührt.

### Schritt (a)

Die Oxidation wird üblicherweise bei erhöhter Temperatur gefahren, um einen hohen Umsatz zu erhalten und um eine Aufpegelung gefährlicher Spurenkomponenten wie Methylnitrat oder Methanthiosulfonsäure-S-methylester, wie sie bei der Herstellung von Methansulfonsäure entstehen können, zu vermeiden. Im allgemeinen wird Schritt (a) bei Reaktionstemperaturen von 50 °C bis 150 °C, bevorzugt von 100 °C bis 140 °C durchgeführt. Der Arbeitsdruck in Schritt (a) liegt im allgemeinen zwischen 100 mbar und 8 bar, bevorzugt bei Normaldruck.

Die in dem erfindungsgemäßen Verfahren eingesetzten Mercaptane und/oder Dialkyldisulfide und/oder Dialkylpolysulfide enthalten Kohlenstoffreste, die aliphatisch oder cycloaliphatisch sein können. Besonders bevorzugt handelt es sich bei den Kohlenwasserstoffresten um lineare oder verzweigte aliphatische Kohlenwasserstoffreste. Diese enthalten vorzugsweise 1 bis 20, besonders bevorzugt 1 bis 14 Kohlenstoffatome. Ganz besonders bevorzugt handelt es sich bei den Resten um Methylreste und somit bei den Alkylmercaptanen oder Dialkyldisulfiden um Methylmercaptan oder Dimethyldisulfid.

Vorzugsweise werden in dem erfindungsgemäßen Verfahren Dialkyldisulfide eingesetzt. Die Dialkyldisulfide werden in der Regel aus Schwefelwasserstoff und Methanol hergestellt, obwohl auch andere Zugangswege in der Literatur bekannt sind. Besonders bevorzugt werden die Dialkyldisulfide durch Oxidation von Alkylmercaptanen mit in einem organischen Dialkyldisulfid gelöstem Schwefel unter Verwendung eines Amins als Katalysator hergestellt. In diesem Verfahren können die Alkylmercaptane als "Roh-Mercaptan-Strom", also als nicht durch Extraktion oder Destillation gereinigter Mercaptan-Strom, aus der Umsetzung von Alkoholen mit Schwefelwasserstoff an einem geeigneten Katalysator eingesetzt werden.

Ein Vorteil dieses Herstellverfahrens für Dialkyldisulfide ist, daß das Verfahren drucklos durchgeführt wird. Das bedeutet, daß zwischengelagertes Dialkyldisulfid nicht in einem Druckbehälter aufbewahrt wird. Zudem stellt Dialkyldisulfid ein lagerstabiles Einsatzmaterial dar und ist somit sicher handhabbar. Dieses Verfahren ist in der gleichzeitig eingereichten Patentanmeldung Nr. 198 54 427.8 (amtliches Aktenzeichen) mit dem Titel "Verfahren zur Herstellung von Dialkyldisulfiden" beschrieben.

Das bevorzugt eingesetze Dialkyldisulfid wird zu Alkansulfonsäure umgesetzt und muß daher nachdosiert werden. Die Nachdosierung von Dialkyldisulfiden kann in die Dampfphase des Reaktionsgemisches in Schritt (a) oder unter die Flüssigkeitsoberfläche des Reaktionsgemisches getaucht erfolgen. Bei Zugabe in die Dampfphase des Reaktionsgemisches können innige Gemische von Dialkyldisulfiden und Stickoxiden entstehen, die explosiv sind. Daher werden die Dialkyldisulfide vorzugsweise unter die Flüssigkeitsoberfläche getaucht zum Reaktionsgemisch zudosiert. Die Tauchung kann beispielsweise im Reaktor über ein Tauchrohr erfolgen oder in einem Umwälzkreis über eine Mischdüse.

Das molare Verhältnis von Alkylmercaptanen und/oder Dialkyldisulfiden und/oder Dialkylpolysulfiden mit drei bis neun Schwefelatomen zu Salpetersäure beträgt bei Mercaptan im allgemeinen 1:1 bis 1:10, bevorzugt 1:2 bis 1:6, besonders bevorzugt 1:2 bis 1:4. Bei Dialkyldisulfiden beträgt das molare Verhältnis im allgemeinen 1:2 bis 1:20, bevorzugt 1:3 bis 1:10, besonders bevorzugt 1:3 bis 1:6.
Die Dialkylpolysulfide werden bevorzugt im Gemisch mit Mercaptanen oder Dialkyldisulfiden eingesetzt.
Die Oxidation kann in einem Reaktor oder in einer Reaktorkaskade mit hohem Rückvermischungsgrad, z.B. in einem Rührkessel oder Schlaufenreaktor, oder in einem Reaktor mit niedrigem Rückvermischungsgrad, z.B. in einem Strömungsrohr, durchgerührt werden. Vorzugsweise wird Schritt (a) in einem Reaktor oder in einer Reaktorkaskade mit hohem Rückvermischungsgrad durchgeführt. Werden Reaktoren oder Reaktorkaskaden mit hohem Rückvermischungsgrad eingesetzt, so können diese wahlweise unterhalb der Siedetemperatur des Reaktionsgemisches als reine Oxidationsreaktoren betrieben werden oder bei der Siedetemperatur des Reaktionsgemisches, wodurch während der Synthese bereits durch Abtrennen von im Überschuß vorhandener, verdünnter wässriger Salpetersäure eine Aufkonzentrierung der Reaktionsmischung erreicht werden kann.

In einer bevorzugten Ausführungsform besteht der Oxidationsteil der Anlage aus einer Reaktorkaskade von zwei Reaktoren mit hohem Rückvermischungsgrad, z.B. zwei Rührkesseln. Die Temperatur im ersten Reaktor, in den Alkylmercaptan bzw. Dialkyldisulfid bzw. Dialkylpolysulfid sowie Salpetersäure dosiert werden, liegt vorzugsweise zwischen 50 und 140 °C, besonders bevorzugt zwischen 80 und 120 °C. Der zweite Reaktor, der mit dem Überlauf des ersten Reaktors beschickt wird, wird vorzugsweise zwischen 100 und 150 °C, besonders bevorzugt zwischen 130 und 150 °C unter Eindampfen des Kesselinhaltes betrieben. Die Verweilzeiten der Reaktionsmischung in beiden Reaktoren können zwischen 10 Minuten und 10 Stunden, bevorzugt zwischen 1 und 3 Stunden liegen.

Ein Teil der Reaktionswärme der Oxidation des Mercaptans bzw. Dialkylsulfids wird vorzugsweise durch einen im Abgasstrom plazierten Kondensator mit Kondensatrückführung zum Reaktionsgemisch abgerührt.

Wird Schritt (a) in einer Reaktorkaskade mit zwei Reaktoren mit hohem Rückvermischungsgrad durchgeführt, so wird im ersten Reaktor das eingesetzte Alkylmercaptan bzw. Dialkyldisulfid bzw. Dialkylpolysulfid weitgehend oxidiert, wobei im wesentlichen die entsprechende Alkansulfonsäure sowie in geringer Menge unvollständige Oxidationsprodukte neben überschüssiger Salpetersäure und geringen Mengen an Schwefelsäure entstehen. Die Ausbeute an Alkansulfonsäure im Gemisch beträgt auf dieser Stufe der Reaktion üblicherweise bereits über 80 %, bevorzugt über 90 %, bezogen auf die eingesetzte Menge Mercaptan und/oder Dialkyldisulfid und/oder Dialkylpolysulfid. In dem zweitem Reaktor erfolgt die Vervollständigung der Oxidationsreaktion, wodurch die Ausbeute an Alkansulfonsäure üblicherweise auf über 90 %, bevorzugt über 93 %, angehoben wird.

Die im Austrag von Reaktionsschritt (a) (mit)enthaltene überschüssige Salpetersäure kann durch einfache Destillation in an sich bekannter Art und Weise mit Wasser destillativ abgetrennt und in die Oxidation von Mercaptanen und/oder Dialkyldisulfiden und/oder Dialkylpolysulfiden (Schritt (a)) oder in die Regenerierung der Stickoxide mit Sauerstoff zu Salpetersäure (Schritt (b)) zurückgeführt werden. Auch die anderen, im Reaktionsaustrag von Schritt (a) entstehenden Nebenprodukte lassen sich destillativ voneinander trennen, wobei die Produkte einer nicht-vollständigen Oxidation vorzugsweise in die Oxidation (Schritt (a)) zurückgerührt werden.

Auf diese Weise wird die Salpetersäure nahezu vollständig im System gehalten, die einzigen Verluste treten über eine sehr geringe Lachgasbildung und eine nicht vollständige Absorption in Schritt (b) auf. Die Absorptionsverluste sind aber nach dem heutigen Stand moderner Salpetersäureanlagen nur gering.

In einer bevorzugten Ausführungsform schließt sich an den zweiten Reaktor eine als Abtriebskolonne betriebene Wasserabtrennkolonne an. Diese trennt als Kopfprodukt Wasser und Salpetersäure ab, als Sumpfprodukt wird typischerweise eine farblose 98 %ige Alkansulfonsäure mit etwa 1 Gew.-% Wasser und etwa 1 Gew.-% Schwefelsäure erhalten. Salpetersäure ist nur in Spuren von < 0,2 Gew.- % enthalten. Die Kolonne wird im allgemeinen bei 20 bis 1000 mbar, bevorzugt bei 50 bis 300 mbar und bei Sumpftemperaturen von im allgemeinen 130 bis 240°C, bevorzugt von 150 bis 200°C betrieben.

### Schritt (b)

Die Regenerierung der Stickoxide (NO/NO₂-Gemische) wird im allgemeinen bei niedrigen Temperaturen und erhöhten Drucken betrieben, um eine möglichst vollständige Absorption der regenerierten Stickoxide NOₓ zu erreichen und so möglichst hoch-konzentrierte Salpetersäure zu erhalten.

Unter NOₓ sind im Sinne der vorliegenden Erfindung im wesentlichen NO, NO₂, N₂O₃, N₂O₄ und N₂O₅ zu verstehen.

Die Konzentration der im erfindungsgemäßen Verfahren eingesetzten Salpetersäure liegt im allgemeinen bei 20 bis 100 Gew.-%, bevorzugt bei 40 bis 70 Gew.-%, besonders bevorzugt bei 50 bis 70 Gew.-%. Vorzugsweise wird Schritt (b) bei Temperaturen von 0 °C bis 60 °C, besonders bevorzugt von 0 °C bis 30 °C isotherm durchgeführt. Die Absolutdrücke betragen vorzugsweise zwischen 0,5 und 20 bar, besonders bevorzugt zwischen 3 und 12 bar.

Die Regenerierung der Stickoxide in Schritt (b) wirkt gleichzeitig als Abgaswäsche für die in Schritt (a) als Nebenprodukte entstehenden Schwefelverbindungen, so daß das Prozeßabgas frei von übelriechenden Mercaptanen oder Dialkyldisulfiden oder Dialkylpolysulfiden ist und in seiner Zusammensetzung in etwa dem gängiger Salpetersäureanlagen entspricht. Somit kann das Abgas ohne zusätzliche Nachbehandlung in die Umgebung abgegeben werden.

Bei dem zur Regenerierung eingesetzten Sauerstoff handelt es sich im allgemeinen um Luftsauerstoff.

Als Reaktionsapparatur wird im allgemeinen eine Absorptionskolonne eingesetzt. Bevorzugt handelt es sich um eine gekühlte Absorptionskolonne, die den bekannten Kolonnen zur Herstellung von Salpetersäure aus Stickoxiden entspricht. Dabei kann es sich zum Beispiel um Siede-, Ventil-, Glocken-, Tunnelbodenkolonnen oder um Kolonnen mit Füllkörpern oder geordneten Packungen handeln. Die Kühlung kann sowohl in der Kolonne als auch in externen Wärmetauschern erfolgen.

Die Absorptionskolonne wird im allgemeinen bei 0 bis 60 °C, bevorzugt bei 0 bis 30 °C betrieben, vorzugsweise isotherm. Am Kopf der Kolonne wird Frischwasser aufgegeben, bevorzugt entsalztes Wasser, dort entweicht eine weitgehend von Stickoxiden befreite Magerluft, d.h. eine an Sauerstoff verarmte Luft.

In dem erfindungsgemäßen Verfahren zur Herstellung von Alkansulfonsäuren wird demgemäß vorzugsweise die in Schritt (a) im Reaktionsaustrag enthaltene Salpetersäure nach Abtrennung aus dem Reaktionsaustrag in Schritt (a) oder Schritt (b) zurückgeführt, und die ebenfalls enthaltenen Produkte einer unvollständigen Oxidation nach Abtrennung in Schritt (a) zurückgeführt.

Die in dem erfindungsgemäßen Verfahren erhaltene, bereits sehr reine Alkansulfonsäure kann in einer nachgeschalteten Vakuumdestillationskolonne, die im allgemeinen bei Kopfdrücken von 0,1 bis 20 mbar, bevorzugt von 2 bis 10 mbar arbeitet, aufgereinigt werden. Hierbei werden in Spuren auftretende Verunreinigungen im Kopf bzw. im Sumpf der Kolonne abgetrennt. Die eigentliche Alkansulfonsäure wird üblicherweise in einem Seitenabzug gewonnen. Die erhaltene Alkansulfonsäure ist farblos und weist im allgemeinen eine Reinheit von > 99 %, bevorzugt von > 99,5 % mit Schwefelsäuregehalt von < 50 ppm auf. Auf diese Weise erhaltene Methansulfonsäure ist z.B. für den Einsatz in galvanischen Bädern geeignet.

Ganz besonders bevorzugt wird in dem erfindungsgemäßen Verfahren Methansulfonsäure durch Oxidation von Dimethyldisulfid hergestellt. Die nach einer Aufreinigung (Vakuumdestillation) erhaltene Methansulfonsäure besitzt im allgemeinen eine Reinheit von > 99 % und ist farblos. Die Schwefelsäuregehalte betragen im allgemeinen weniger als 50 ppm. Eine solche Methansulfonsäure ist besonders für den Einsatz in galvanischen Bädern geeignet.

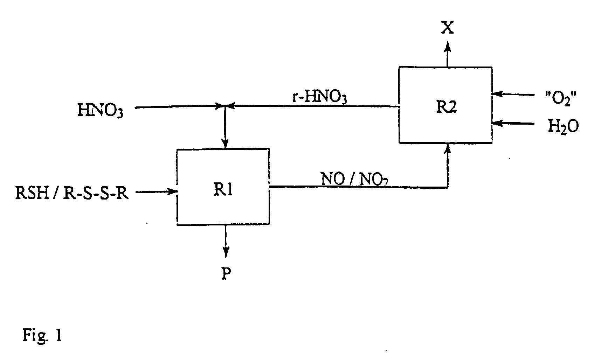

In der anliegenden Zeichnung ist in Fig. 1 das erfindungsgemäße Verfahren schematisch dargestellt.

Darin bedeuten:
- R1: Reaktor 1, in dem Schritt (a) durchgeführt wird
- R2: Reaktor 2, in dem Schritt (b) durchgeführt wird
- RSH/R-S-S-R: eingesetztes Mercaptan und/oder eingesetztes Dialkyldisulfid
- HNO₃: eingesetzte Salpetersäure
- r-HNO₃: aus Schritt (b) in Schritt (a) rückgeführte Salpetersäure
- NO/NO₂: niedrig oxidierte Stickstoffverbindungen (NO/NO₂-Gemische)
- H₂O: Wasser
- "O₂": Luftsauerstoff
- X: Abgas
- P: Reaktionsaustrag, enthaltend das Reaktionsprodukt

Das nachfolgende Beispiel erläutert die Erfindung zusätzlich.

### Beispiel

### Versuchsaufbau:

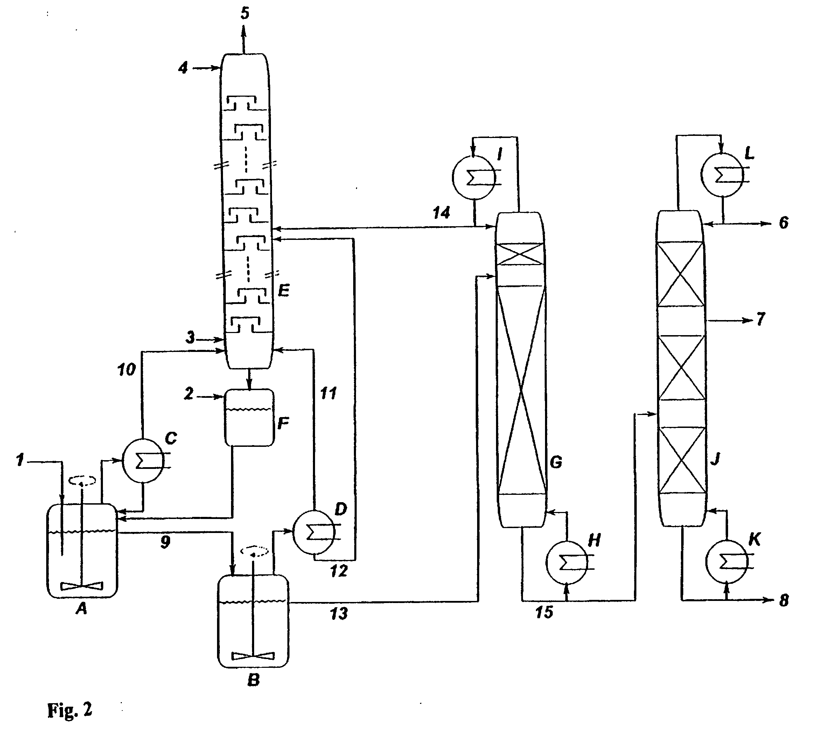

Das beiliegende Anlagenschema (Figur 2) gibt den Versuchsaufbau wieder.

### Apparate:

- A: 1. Oxidationsreaktor (Rührkessel)
- B: 2. Oxidationsreaktor (Rührkessel)
- C: Kondensator
- D: Kondensator
- E: Bodenkolonne mit 44 Glockenböden zur Salpetersäureregeneration
- F: Pufferbehälter für Salpetersäure
- G: 1. Vakuumrektifikationskolonne mit Glasringschüttung
- H: Sumpfwärmetauscher
- I: Kondensator mit Rücklaufteiler
- J: 2. Vakuumrektifikationskolonne mit geordneten Packungen
- K: Sumpfwärmetauscher
- L: Kondensator mit Rücklaufteiler

### Ströme:

- 1: Zulauf an reinem Dimethyldisulfid
- 2: Salpetersäurezulauf
- 3: Luftzufuhr
- 4: Zulauf von entionisiertem Wasser
- 5: Abluft
- 6: Leichtsiederablauf
- 7: Methansulfonsäureablauf
- 8: Hochsiederablauf
- 9: Ablauf vom 1. zum 2. Oxidationsreaktor
- 10: Abgas vom 1. Oxidationsreaktor
- 11: Abgas vom 2. Oxidationsreaktor
- 12: Kondensatstrom vom 2. Oxidationsreaktor zur Salpetersäureregeneration
- 13: Ablauf vom 2. Oxidationsreaktor zur 1. Vakuumrektifikationskolonne
- 14: Kondensatstrom von der 1. Vakuumrektifikationskolonne zur Salpetersäureregeneration
- 15: Sumpfaustrag von der 1. zur 2. Vakuumrektifikationskolonne

### Versuchsdurchführung:

Der Reaktor A wird unter Rühren kontinuierlich über 1 mit reinem Dimethyldisulfid (> 98 %) und aus F mit 45 bis 50 %iger Salpetersäure im Molverhältnis DMDS (Dimethyldisulfid) : HNO₃ 1 : 5 beschickt. Die Zuführung des Dimethyldisulfids erfolgt getaucht. Die Temperatur im Reaktor A liegt bei 100°C. Die Verweilzeit im Reaktor A, berechnet als Quotient des Flüssigkeitsvolumens im Reaktor A, geteilt durch den den Reaktor A kontinuierlich verlassenden Flüssigstrom 9, liegt bei ca. 2,2 h.
Der den Reaktor A kontinuierlich verlassende Flüssigkeitsstrom 9 besteht aus ca. 32 % Methansulfonsäure, 11 % Salpetersäure, 0,6 % Methanthiosulfonsäure-S-methylester und 56 % Wasser und wird dem Reaktor B zugeführt.

Die Temperatur im Reaktor B liegt bei 130°C. Die Verweilzeit im Reaktor B, berechnet als Quotient des Flüssigkeitsvolumens im Reaktor B, geteilt durch den den Reaktor B kontinuierlich verlassenden Flüssigstrom 13, liegt bei ca. 2,2 h.
Der den Reaktor B kontinuierlich verlassende Flüssigkeitsstrom 13 besteht aus ca. 55 % Methansulfonsäure, 10 % Salpetersäure, < 0,2 % Methanthiosulfonsäue-Smethylester und 35 % Wasser und wird der Vakuumrektifikationskolonne G knapp unterhalb des Kolonnenkopfes zugeführt. Die Rohausbeute an Methansulfonsäure im Strom 9 liegt bei > 95 %.

Die Vakuumrektifikationskolonne G arbeitet bei 95 bis 100 mbar Kopfdruck (absolut) und 180 bis 190°C Sumpftemperatur.

Der die Vakuumrektifikationskolonne G verlassende Sumpfaustrag 15 besteht aus ca. 98 % Methansulfonsäure, ca. 1 % Wasser und ca. 1 % Schwefelsäure und wird der Vakuumrektifikationskolonne J zugeführt.

Die Vakuumrektifikationskolonne J arbeitet bei ca. 5 bis 10 mbar Kopfdruck (absolut) und ca. 180 bis 190°C Sumpftemperatur.

Der die Vakuumrektifikationskolonne J verlassende Seitenbabzugsstrom 7 besteht aus > 99 %iger Methansulfonsäure mit einem Schwefelsäuregehalt von < 50 ppm. Die Gesamtausbeute an Methansulfonsäure nach Destillation liegt bei > 90 %.

Der die Vakuumrektifikationskolonne J verlassende Kopfaustragsstrom 6 besteht aus Wasser, Methansulfonsäure, Methansulfonsäuremethylester und anderen Leichtsiedern. Der die Vakuumrektifikationskolonne J verlassende Sumpfaustragsstrom 8 besteht aus Schwefelsäure, Methansulfonsäure und anderen Hochsiedern.

Die Bodenkolonne zur Salpetersäureregeneration E arbeitet bei Normaldruck und bei Temperaturen von 20 bis 45°C.
Der Bodenkolonne zur Salpetersäureregeneration E wird über den Zulauf 4 entionisiertes Wasser zugeführt.
Die der Bodenkolonne zur Salptetersäureregeneration E über den Strom 3 zugeführte Luft zur Reoxidation der Stickoxide verläßt die Kolonne am Kopfausgang 5 mit vermindertem Sauerstoffgehalt (7 bis 13 Vol%).
Der im Reaktor A gebildete und im Kondensator C von kondensierbaren Komponenten befreite NOₓ-haltige Abgasstrom 10 enthält NO und NO₂ und wird der Bodenkolonne zur Salpetersäureregeneration E zugeführt.
Der im Reaktor B gebildete und im Kondensator D von kondensierbaren Komponenten befreite NOₓ-haltige Abgasstrom 11 enthält NO und NO₂ und wird der Bodenkolonne zur Salpetersäureregeneration E zugeführt.
Das Kondensat 12 besteht aus ca. 7 %iger Salpetersäure und wird der Bodenkolonne zur Salpetersäureregeneration E an einem Boden mit ähnlicher Bodenkonzentration an Salpetersäure zugeführt.

Das Kondensat 14 besteht aus ca. 23 %iger Salpetersäure und wird der Bodenkolonne zur Salpetersäureregeneration E an einem Boden mit ähnlicher Bodenkonzentration an Salpetersäure zugeführt.
Der Sumpfablauf der Bodenkolonne zur Salpetersäureregeneration E zum Salpetersäurepufferbehälter F besteht aus ca. 45 bis 50%iger Salpetersäure und wird dem Reaktor A zugeführt.
Salpetersäureverluste werden durch Nachfüllen der erforderlichen Mengen an 50 bis 65 %iger Salpetersäure über den Strom 2 in den Salpetersäurepufferbehälter F ersetzt.

## Patentansprüche

1. Verfahren zur Herstellung von Alkansulfonsäure, umfassend folgende Schritte:
(a) Oxidation von Alkylmercaptanen und/oder Dialkyldisulfiden und/oder Dialkylpolysulfiden mit drei bis neun Schwefelatomen mit Salpetersäure unter Bildung von Alkansulfonsäuren, Wasser, Stickoxiden sowie weiteren Nebenprodukten,
(b) Regenerierung der aus Schritt (a) erhaltenen Stickoxide mit Sauerstoff zu Salpetersäure und Rückführung der Salpetersäure in Schritt (a),
**dadurch gekennzeichnet, daß** die Schritte (a) und (b) in voneinander getrennten Reaktionsräumen durchgeführt werden.

2. Verfahren nach Anspruch 1, **dadurch gekennzeichnet, daß** das Verfahren kontinuierlich durchgeführt wird.

3. Verfahren nach Anspruch 1 oder 2, **dadurch gekennzeichnet, daß** Schritt (a) bei Reaktionstemperaturen von 50 bis 150 °C und bei einem Arbeitsdruck von 100 mbar bis 8 bar durchgeführt wird.

4. Verfahren nach einem der Ansprüche 1 bis 3, **dadurch gekennzeichnet, daß** die Alkylmercaptane oder die Dialkyldisulfide oder die Dialkylpolysulfide Kohlenwasserstoffreste mit 1 bis 20 Kohlenstoffatomen enthalten.

5. Verfahren nach einem der Ansprüche 1 bis 4, **dadurch gekennzeichnet, daß** in Schritt (a) Dialkyldisulfide oxidiert werden.

6. Verfahren nach Anspruch 5, **dadurch gekennzeichnet, daß** Dimethyldisulfid oxidiert wird.

7. Verfahren nach einem der Ansprüche 1 bis 6, **dadurch gekennzeichnet, daß** ein Teil der Reaktionswärme der Oxidation des Mercaptans bzw. des Dialkyldisulfids bzw. des Dialkylpolysulfids durch einen im Abgasstrom plazierten Kondensator mit Kondensatrückführung abgeführt wird.

8. Verfahren nach einem der Ansprüche 1 bis 7, **dadurch gekennzeichnet, daß** Schritt (b) bei Temperaturen von 0 bis 60 °C und bei einem Arbeitsdruck von 0,5 bis 20 bar durchgeführt wird.

9. Verfahren nach einem der Ansprüche 1 bis 8, **dadurch gekennzeichnet, daß** Schritt (b) in einer gekühlten Absorptionskolonne durchgeführt wird.

10. Verfahren nach einem der Ansprüche 1 bis 9, **dadurch gekennzeichnet, daß** die in Schritt (a) im Reaktionsaustrag enthaltene Salpetersäure nach Abtrennung aus dem Reaktionsgemisch in Schritt (a) oder Schritt (b) zurückgeführt wird, und die ebenfalls enthaltenen Produkte einer unvollständigen Oxidation nach Abtrennung in Schritt (a) zurückgeführt werden und die Alkansulfonsäure abgetrennt wird.

## Claims

1. A process for the preparation of alkanesulfonic acids, comprising the following steps:
(a) oxidation of alkylmercaptans and/or dialkyl disulfides and/or dialkyl polysulfides having from three to nine sulfur atoms with nitric acid to form alkanesulfonic acids, water, nitrogen oxides and other byproducts,
(b) regeneration of the nitrogen oxides obtained from step (a) with oxygen to give nitric acid and recycling of the nitric acid to step (a),
which comprises carrying out steps (a) and (b) in reaction chambers separate from one another.

2. A process as claimed in claim 1, wherein the process is carried out continuously.

3. A process as claimed in claim 1 or 2, wherein step (a) is carried out at reaction temperatures of from 50 to 150°C and at an operating pressure of from 100 mbar to 8 bar.

4. A process as claimed in any of claims 1 to 3, wherein the alkyl mercaptans or the dialkyl disulfides or the dialkyl polysulfides contain hydrocarbon radicals having from 1 to 20 carbon atoms.

5. A process as claimed in any of claims 1 to 4, wherein dialkyl disulfides are oxidized in step (a) .

6. A process as claimed in claim 5, wherein dimethyl disulfide is oxidized.

7. A process as claimed in any of claims 1 to 6, wherein some of the heat of the reaction of the oxidation of the mercaptan or of the dialkyl disulfide or of the dialkyl polysulfide is dissipated by a condenser placed in the offgas stream with condensate recycling.

8. A process as claimed in any of claims 1 to 7, wherein step (b) is carried out at from 0 to 60°C and at an operating pressure of from 0.5 to 20 bar.

9. A process as claimed in any of claims 1 to 8, wherein step (b) is carried out in a cooled absorption column.

10. A process as claimed in any of claims 1 to 9, wherein the nitric acid present in the reaction discharge in step (a) is, after having been removed from the reaction mixture, returned to step (a) or step (b), and the products of incomplete oxidation which are likewise present are, after they have been removed, returned to step (a), and the alkanesulfonic acid is separated off.

## Revendications

1. Procédé pour la fabrication d'acide sulfonique d'alcane, comprenant les étapes suivantes :
(a) oxydation d'alkylmercaptans et/ou de bisulfures de dialkyle et/ou de polysulfures de dialkyle avec de trois à neuf atomes de soufre avec de l'acide nitrique, avec formation d'acides sulfoniques d'alcane, d'eau, d'oxydes nitriques ainsi que d'autres sous-produits,
(b) régénération des oxydes nitriques obtenus à l'étape (a) avec de l'oxygène pour obtenir de l'acide nitrique et réintroduction de l'acide nitrique à l'étape (a),
**caractérisé en ce que** les étapes (a) et (b) sont réalisées dans des espaces de réaction séparés.

2. Procédé selon la revendication 1, **caractérisé en ce que** le procédé est réalisé en continu.

3. Procédé selon la revendication 1 ou 2, **caractérisé en ce que** l'étape (a) est réalisée à des températures de réaction de 50 à 150 °C et avec une pression de service de 100 mbar à 8 bar.

4. Procédé selon une des revendications 1 à 3, **caractérisé en ce que** les alkylmercaptans, les bisulfures de dialkyle ou les polysulfures de dialkyle contiennent des résidus d'hydrocarbures avec 1 à 20 atomes de carbone.

5. Procédé selon une des revendications 1 à 4, **caractérisé en ce que** les bisulfures de dialkyle sont oxydés à l'étape (a).

6. Procédé selon la revendication 5, **caractérisé en ce que** du bisulfure diméthyle est oxydé.

7. Procédé selon une des revendications 1 à 6, **caractérisé en ce qu'**une partie de la chaleur de réaction de l'oxydation du mercaptan, du bisulfure de dialkyle ou du polysulfure de dialkyle est évacuée par un condensateur placé dans le flux de gaz d'échappement avec réintroduction des condensats.

8. Procédé selon une des revendications 1 à 7, **caractérisé en ce que** l'étape (b) est réalisée à des températures de 0 à 60 °C et avec une pression de service de 0,5 à 20 bar.

9. Procédé selon une des revendications 1 à 8, **caractérisé en ce que** l'étape (b) est réalisée dans une colonne d'absorption refroidie.

10. Procédé selon une des revendications 1 à 9, **caractérisé en ce que** l'acide nitrique contenu dans le produit de la réaction de l'étape (a) est réintroduit après extraction du mélange de réaction dans l'étape (a) ou l'étape (b), les produits également contenus d'une oxydation incomplète sont réintroduits après extraction dans l'étape (a) et l'acide sulfonique d'alcane est extrait.
